# EUROPÄISCHE PATENTSCHRIFT

(11) **EP 2 729 199 B1**
(45) Veröffentlichungstag und Bekanntmachung des Hinweises auf die Patenterteilung: **27.06.2018**
(21) Anmeldenummer: 12740082.8
(22) Anmeldetag: 29.06.2012
(51) Int. Cl.: A61M 1/36

(54) **DIALYSEVERFAHREN ZUR ENTFERNUNG PROTEINGEBUNDENER TOXINE AUS DEM BLUT VON AKUT ODER CHRONISCH-NIERENINSUFFIZIENTEN PATIENTEN**
DIALYSIS METHOD FOR REMOVING PROTEIN-BONDED TOXINS FROM THE BLOOD OF PATIENTS HAVING ACUTE OR CHRONIC RENAL INSUFFICIENCY
PROCÉDÉ DE DIALYSE POUR EXTRAIRE, DU SANG DE PATIENTS SOUFFRANT D'INSUFFISANCE RÉNALE AIGUË OU CHRONIQUE, DES TOXINES LIÉES AUX PROTÉINES

(30) Priorität: 05.07.2011 DE 102011078700
(43) Veröffentlichungstag der Anmeldung: 14.05.2014
(73) Patentinhaber: 3M Innovative Properties Company, St. Paul, MN 55133-3427 (US)
(72) Erfinder: JANKOWSKI, Joachim, 14532 Stahnsdorf (DE); ZIDEK, Walter, 14167 Berlin (DE); BRETTSCHNEIDER, Falko, 14052 Berlin (DE); LEMKE, Horst-Dieter, 63785 Obernburg (DE)
(74) Vertreter: Gulde & Partner
(86) Internationale Anmeldenummer: PCT/EP2012/062658
(87) Internationale Veröffentlichungsnummer: WO 2013/004605

(56) Entgegenhaltungen:
- WO-A1-91/06326
- WO-A1-98/52628
- US-A- 4 581 141
- US-A1- 2008 015 487
- US-A1- 2010 096 329

## Beschreibung

Primäre Aufgabe der Nieren ist die Ausscheidung harnpflichtiger Substanzen, den sogenannten Urämie-Toxinen. Nieren chronisch-niereninsuffizienter Patienten können diese Aufgaben nicht mehr erfüllen, was im unbehandelten Zustand rasch zur Vergiftung des Patienten und damit zum Tod führt. Die Dialyse ist das Instrument der Wahl zur Linderung der akuten und chronischen Erkrankung und zur zeitlichen Überbrückung bis ein geeignetes Spenderorgan zur Verfügung steht. Die Dialyse beruht auf dem Prinzip des Stoffaustauschs durch Filtration bzw. Diffusion. Dabei wirken die derzeit verwendeten Membranen als reine Filter- und/oder Diffusionsmembranen und sorgen dafür, dass dem zu reinigenden Blut Stoffe entzogen werden, die eine bestimmte Größe nicht überschreiten. Derzeit gebräuchliche High-flux Dialysemembranen haben eine Ausschlussgrenze von beispielsweise 14.000-17.000 Da. Durch die derzeit angewandten Dialysetechniken ist aber im Regelfall eine vollständige Abtrennung der Urämie-Toxine nicht möglich, da ein Teil der harnpflichtigen Substanzen proteingebundenen vorliegen. Hierbei handelt sich unter anderem um niedermolekulare, aromatische Substanze. Urämie-Toxine, die in der Regel proteingebunden vorliegen können, sind beispielsweise Phenylessigsäure, p-Cresylsulfat, p-Hydroxyhippursäure und Indoxylsulfat. In der Folge akkumulieren die betreffenden Substanzen im Organismus des Patienten und bedingen dort Folgeerkrankungen der akuten und chronischen Niereninsuffizienz. Bei chronisch niereninsuffizienten Patienten treten dadurch bedingt zunehmend Folgeerkrankungen wie Herzkreislauferkrankungen, und daraus resultierend eine erhöhte Sterblichkeitsrate auf. Die Dialyse beschleunigt z. B. die Entstehung von Arteriosklerose. Gefäßerkrankungen wie Arteriosklerose verursachen die häufigsten Todesfälle bei dieser Patientengruppe.

Ein Grund dafür sind niedermolekulare, hydrophobe und aromatische Urämie-Toxine. Aromatische, hydrophobe Urämie-Toxine weisen eine geringe Wasserlöslichkeit auf. Daher kommt es zwischen diesen Substanzen und Plasmaproteinen meist zur Ausbildung adsorptiver Effekte. Diese adsorptiven Effekte gehen auf unterschiedliche Wechselwirkungen zurück. Hierbei sind vor allem Wasserstoffbrückenbindungen, lonenbindungen und Dipol-Dipol-Wechselwirkungen (van-der-Waals-Kräfte) zu nennen. Durch die Bindung an Plasmaproteine, wie z. B. an Albumin, kann das effektive Molekulargewicht der harnpflichtigen Substanzen derart erhöht werden, dass effektive Molekulargewichte von deutlich größer als 17.000 Da erreicht werden. Das kombinierte Molekulargewicht der proteingebundenen Urämie-Toxine liegt dann meist oberhalb der Ausschlussgrenze der verwendeten Dialysemembranen und diese können somit bei der Dialyse nicht effektiv entfernt werden.

Durch die Dialyse kann folglich nur der nicht-proteingebundene Anteil des betreffenden Urämie-Toxins abgetrennt werden. Der proteingebundene Anteil (bis zu 95 % der Gesamtmenge des Urämie-Toxins) wird dadurch nicht grundlegend verändert. Aufgrund des Gleichgewichts gemäß des Massenwirkungsgesetz zwischen proteingebundenen und nicht-proteingebundenen Urämie-Toxinen werden unmittelbar nach der Dialyse wieder im Wesentlichen die initialen, nicht-proteingebundene Urämie-Toxinkonzentrationen im Plasma chronisch-niereninsuffizienter Patienten erreicht. Da die pathophysiologischen und pathochemischen Wirkungen im Besonderen von nicht-proteingebundenen Urämie-Toxinen bedingt werden, wird durch diese Gleichgewichtseinstellung ein für die Patienten fataler Prozess initiiert. Dieser *circulus vitiosus* ist Ursache für die vielfältigen pathologischen Erscheinungsformen der chronischen Niereninsuffizienz. Bis zum heutigen Tag sind keine konventionellen Methoden verfügbar, durch die auch proteingebundene Urämie-Toxine während der Dialyse effektiv aus dem zu reinigenden Blut entfernt werden können.

Der vorliegenden Erfindung liegt die Aufgabe zugrunde mindestens einen Nachteil des geschilderten Standes der Technik zu vermindern oder zu vermeiden. Insbesondere ist es eine Aufgabe der Erfindung Mittel und Wege zur Verfügung zu stellen, mit denen proteingebundene Urämie-Toxine effektiv aus dem Blut von Dialysepatienten entfernt werden können.

Die Aufgabe wird gelöst durch die in den Ansprüchen definierten Gegenstände.

Insbesondere offenbart die vorliegende Erfindung eine Verwendung von vorzugsweise natürlicherweise in Blutplasma vorkommenden Ionen oder Salzen davon in einem Verfahren zur Dialyse mittels eines Dialysators zum Stoffaustausch, insbesondere zur Hämodialyse oder Hämodiafiltration, dadurch gekennzeichnet, dass dem zu reinigenden Blut vor dem Beginn und/oder während der Passage durch den Dialysator ein oder mehrere natürlicherweise in Blutplasma vorkommende Ionen oder Salze davon zugesetzt werden, so dass das zu reinigende Blut zu Beginn und/oder während der Passage durch den Dialysator bzw. im Verlaufe des Dialysatordurchlaufs mindestens in Bezug auf eines dieser Ionen oder Salze davon eine Konzentration aufweist, die gegenüber der physiologischen Konzentration des Ions oder Salzes in Blutplasma erhöht ist.

Der Erfindung liegt die Erkenntnis zugrunde, dass die Bindungen zwischen bestimmten Urämie-Toxinen und Plasmaproteinen keine kovalenten chemischen Bindungen sind, sondern es sich um reversible hydrophobe oder/und ionische Bindungen handelt. Diese Bindungen beruhen im Wesentlichen auf den elektrostatischen Eigenschaften und Wechselwirkungen der beteiligten Moleküle. Es wurde gefunden, dass die Stärke dieser Bindungen oder Wechselwirkungen erfindungsgemäß durch die Erhöhung einer lonenkonzentration über eine physiologische Konzentration hinaus herabgesetzt werden kann. Durch diesen Schritt kann während der Dialyse der Anteil proteingebundener Urämie-Toxine deutlich reduziert werden. Im Rahmen der konventionellen, im klinischen Alltag durchgeführten Dialyse kann durch zusätzlichen Eintrag von natürlicherweise im Blutplasma vorkommenden Ionen oder Salzen davon vor der Passage des zu reinigenden Blutes durch den Dialysator die Freisetzungsrate protein-gebundener Urämie-Toxine aus der Proteinbindung erhöht werden. Dadurch wird während der Dialyse eine vermehrte Freisetzung proteingebundener urämischer Toxine erreicht und damit in Folge eine verbesserte Abtrennung dieser Substanzen aus dem Blut des Patienten erreicht. Die betreffenden Urämie-Toxine können somit vermehrt und effektiv dialysiert werden. Die zusätzlich dem zu reinigenden Blut zugesetzten Ionen oder Salze davon werden im Dialysator ebenfalls effektiv aus dem Blut dialysiert, so dass das Blut nach der Passage durch den Dialysator in Bezug auf alle natürlicherweise in Blutplasma vorkommenden Ionen oder Salzen davon eine physiologische Konzentration oder mindestens eine tolerierbare Konzentration aufweist und dem Patienten ohne unzumutbare Gefährdung zurückgeführt werden kann.

In dem Dialyseverfahren der erfindungsgemäßen Verwendung wird ein Dialysator zum Stoffaustausch verwendet. Aufgabe des Dialysators ist es, dem zu reinigenden Blut Urämie-Toxine möglichst effektiv zu entziehen. Im Dialysator sind zu reinigendes Blut und eine Flüssigkeit, gegen die dialysiert werden soll, das sog. Dialysat, durch eine semipermeable Membran voneinander getrennt. In der Regel wird im Dialysator das Dialysat in einem Dialysatkreislauf im Gegenstrom zum Blutfluss im Blutkreislauf geführt. Grundsätzlich ist eine Führung von Blut und Dialysat durch den Dialysator im Gleichstrom auch möglich allerdings ist eine solche Anordnung in der Regel weniger effektiv als eine Anordnung im Gegenstromprinzip. Über die semipermeable Membran des Dialysators erfolgt der Stoffaustausch zwischen Blut auf der einen Seite und Dialysat auf der anderen Seite der Membran. Dabei erfolgt der Stofftransport der Urämie-Toxine über die Membran durch Diffusion (Hämodialyse) oder Diffusion und Konvektion (Hämodiafiltration). Die Selektivität des Stoffaustausches wird einerseits durch die Eigenschaften der Membran, wie z. B. die Porengröße, bestimmt, andererseits durch die Zusammensetzung des Dialysats. Geeignete Dialysatoren sind im Stand der Technik beschrieben und deren Verwendung ist dem Fachmann bekannt. Üblicherweise werden Kapillardialysatoren eingesetzt. Der Dialysator umfasst bevorzugt eine semipermeable High-flux Membran mit einer Größenausschlussgrenze von ≥ 10.000 Da, bevorzugt einer Größenausschlussgrenze die ausgewählt ist aus dem Bereich von 10.000 bis 20.000 Da, besonders bevorzugt von 14.000 bis 17.000 Da. Insbesondere kann ein Dialysator zum Einsatz kommen, der eine semipermeable Membran aufweist mit einer Größenausschlussgrenze, die geeignet ist, die vor dem Beginn und/oder während der Passage durch den Dialysator dem zu reinigenden Blut zugesetzten Ionen oder Salze durch die Membran passieren zu lassen, bevorzugt mit einer Größenausschlussgrenze von ≥ 10.000 Da.

Im Rahmen der erfindungsgemäßen Verwendung wird das zu reinigende Blut vor dem Beginn der Passage durch den Dialysator und/oder während der Passage durch den Dialysator mit einem oder mehreren natürlicherweise in Blutplasma vorkommenden Ionen oder Salzen davon versetzt, so dass das zu reinigende Blut zu Beginn und/oder während der Passage durch den Dialysator mindestens in Bezug auf eines dieser Ionen oder Salze davon eine Konzentration aufweist, die gegenüber der physiologischen Konzentration des betreffenden Ions oder Salzes in Blutplasma erhöht ist. Dabei wird das zu reinigende Blut in der Regel nach der Entnahme aus dem Patienten und vor dem Beginn und/oder während der Passage durch den Dialysator mit Ionen oder Salzen versetzt. Eine Verabreichung dieser Ionen oder Salze vor der Entnahme des Blutes aus dem Patienten scheidet allein schon deswegen aus, weil damit in Bezug auf diese Ionen oder Salze gegenüber der physiologischen Konzentration erhöhte Konzentrationen im Patienten erreicht werden würden, die ggf. gesundheitsgefährdend sein können.

Werden dem zu reinigenden Blut vor und/oder während der Passage durch den Dialysator mehr als ein natürlicherweise in Blutplasma vorkommendes Ion oder Salz davon zugesetzt, so können diese Ionen oder Salze davon in einer Konzentration zugesetzt werden, so dass das zu reinigende Blut zu Beginn der Passage und/oder während der Passage durch den Dialysator in Bezug auf eines, mehrere oder alle zugesetzten Ionen oder Salze davon eine Konzentration aufweist, die gegenüber der physiologischen Konzentration der Ionen oder Salze in Blutplasma erhöht ist.

Als gegenüber der physiologischen Konzentration in Blutplasma erhöht gilt die Konzentration eines Ions oder Salzes davon insbesondere dann, wenn die Konzentration des betreffenden Ions oder Salzes davon im zu reinigenden Blut zu Beginn und/oder während der Passage des Dialysators eine Konzentration aufweist, die gegenüber der physiologischen Konzentration des Ions oder Salzes davon in Blutplasma mindestens um einen Faktor 1,2 erhöht ist, bevorzugt um einen Faktor ≥ 2, besonders bevorzugt um einen Faktor ≥ 1,5 bis 10, ganz besonders bevorzugt von ≥ 2 bis 8.

Bei den natürlicherweise in Blutplasma vorkommenden Ionen oder Salzen davon, die erfindungsgemäß verwendet werden können, handelt es sich um das Ion Na⁺ oder das Salz NaCl.

Als physiologische Konzentration eines natürlicherweise in Blutplasma vorkommenden Ions wird eine Konzentration angesehen, die sich in einem Bereich bewegt, in dem das betreffende Ion üblicherweise bei gesunden Menschen nachgewiesen wird. Im Folgenden sind Beispiele für den Bereich einer physiologischen Konzentration für ausgewählte natürlicherweise in Blutplasma vorkommende Ionen dargestellt:

| Ion | physiologische Konzentration |
|---|---|
| Na⁺ | 136 bis 146 mmol/l |
| K⁺ | 3,8 bis 5,2 mmol/l |
| Ca²⁺ | 2,3 bis 2,7 mmol/l |
| Mg²⁺ | 0,8 bis 1,2 mmol/l |
| Cl⁻ | 96 bis 106 mmol/l |
| HCO₃⁻ | 24 bis 28 mmol/l |
| PO₄³⁻ | 1,0 bis 1,4 mmol/l |
| Glycin | 250 µmol/l |

Bevorzugt handelt es sich bei einer physiologischen Konzentration um die höhere Grenze des für das jeweilige Ion angegebenen physiologischen Konzentrationsbereichs.

Erfindungsgemäß wird dem zu reinigenden Blut vor dem Beginn und/oder während der Passage durch den Dialysator das Ion Na⁺ oder das Salz NaCl zugesetzt, so dass das zu reinigende Blut zu Beginn und/oder während mindestens eines Teils der Passage durch den Dialysator wenigstens in Bezug auf dieses Ion oder Salz eine Konzentration aufweist, die gegenüber der physiologischen Konzentration dieses Ions oder Salzes in Blutplasma erhöht ist. Insbesondere kann dem zu reinigenden Blut das Ion Na⁺ oder das Salz NaCl derart zugesetzt werden, dass das zu reinigende Blut zu Beginn und/oder während der Passage durch den Dialysator eine Na⁺- oder eine NaCI-Konzentration aufweist von ≥ 0,25 M, bevorzugt von ≥ 0,3 M, besonders bevorzugt von ≥ 0,25 M bis 1,5 M, ganz besonders bevorzugt von ≥ 0,3 M bis 1 M.

Das zu reinigende Blut wird bevorzugt nach der Entnahme aus dem Patienten und vor dem Beginn der Passage durch den Dialysator mit natürlicherweise in Blutplasma vorkommenden Ionen oder Salzen davon versetzt. D. h. zum Zeitpunkt des ggf. ersten Kontaktes zwischen zu reinigendem Blut und semipermeabler Membran des Dialysators liegt mindestens eines dieser Ionen oder Salze im zu reinigenden Blut in einer Konzentration vor, die gegenüber der physiologischen Konzentration des betreffenden Ions oder Salzes erhöht ist. Dies hat den Vorteil, dass die Lösung der Urämie-Toxine aus der Proteinbindung bereits zu Beginn der Passage durch den Dialysator erfolgt und somit die volle Kapazität des Dialysators für den Stoffaustausch mit dem Dialysat zur Verfügung steht.

Um das zu reinigende Blut vor dem Beginn und/oder während der Passage durch den Dialysator mit einem natürlicherweise in Blutplasma vorkommenden Ion oder Salz davon zu versetzen, kann das zu reinigende Blut beispielsweise mit einer Lösung versetzt werden, die das betreffende Ion oder Salz davon in geeigneter Konzentration enthält. Die tatsächliche Konzentration des Ions oder Salzes in dieser Lösung hängt von verschiedenen Parametern ab. Die Konzentration in der Lösung, also die Ausgangskonzentration, hängt beispielsweise davon ab, in welcher Konzentration das betreffende Ion oder Salz im zu reinigenden Blut zu Beginn und/oder während der Passage durch den Dialysator vorliegen soll, also von der Zielkonzentration, und welche Flussraten im Blutkreislauf und bei der Zudosierung der betreffenden Lösung herrschen sollen. Die Flussraten können im Einzelfall variieren und hängen sowohl von individuellen Parametern der Patienten ab als auch von gerätespezifischen Parametern oder gewünschten Diffusions- und/oder Konvektionsraten. Geeignete Ausgangskonzentrationen in der Lösung und geeignete Flussraten können durch den Fachmann zuverlässig und ohne unzumutbaren Aufwand für den Einzelfall bestimmt werden.

Üblicherweise werden in der Hämodialyse und der Hämodiafiltration Dialysat- oder Substitutionslösungen eingesetzt, die insbesondere in Bezug auf ihre lonen- oder Salzzusammensetzung physiologische Konzentrationen aufweisen. Solche physiologischen Elektrolytlösungen können auch zum Eintrag von natürlicherweise in Blutplasma vorkommenden Ionen oder Salzen davon bei der erfindungsgemäßen Verwendung eingesetzt werden, wobei die physiologische Elektrolytlösung mindestens ein natürlicherweise in Blutplasma vorkommendes Ion oder Salz davon in einer Konzentration enthalten muss, die gegenüber der physiologischen Konzentration des betreffenden Ions oder Salzes erhöht ist. Insbesondere kann das zu reinigende Blut nach der Entnahme und vor dem Beginn und/oder während der Passage durch den Dialysator mit einer Elektrolytlösung versetzt werden, die in Bezug auf ein oder mehrere natürlicherweise in Blutplasma vorkommende Ionen oder Salze davon eine Konzentration aufweist, die gegenüber der physiologischen Konzentration der betreffenden Ionen oder Salze in Blutplasma erhöht ist, während die Elektrolytlösung zusätzlich andere natürlicherweise in Blutplasma vorkommende Ionen oder Salze davon in physiologischer Konzentration enthalten kann.

Die Entfernung der Urämie-Toxine aus dem zu reinigenden Blut erfolgt während der Passage durch den Dialysator durch Diffusion oder Konvektion und Diffusion an der semipermeablen Membran des Dialysators. Die semipermeable Membran des Dialysators ist permeabel für die natürlicherweise in Blutplasma vorkommenden Ionen oder Salze, sodass im Dialysator nicht nur eine effektive Entfernung von Urämie-Toxinen erfolgt sondern gleichzeitig auch überschüssige Mengen an vorher zugeführten Ionen oder Salzen dialysiert werden. Damit weist das gereinigte Blut nach der Passage durch den Dialysator für alle natürlicherweise in Blutplasma vorkommenden Ionen oder Salze davon im Wesentlichen wieder eine physiologische Konzentration auf oder wenigstens eine Konzentration, die für den Patienten tolerierbar ist. Im Rahmen der erfindungsgemäßen Verwendung kann vorgesehen sein, dass bestimmte Parameter des Blutes vor der Rückführung des Blutes in den Patienten gemessen und auf Überschreitung von Grenzwerten hin überprüft werden. So kann insbesondere vorgesehen sein, die Rückführung des gereinigten Blutes nur zu erlauben solange ein vorher festgelegter Grenzwert für einen solchen gemessenen Parameter nicht überschritten worden ist. Ein Beispiel für einen solchen Parameter ist die Leitfähigkeit des Blutes. Wird bei dem gereinigten Blut nach der Passage durch den Dialysator und vor der Rückführung in den Patienten eine Leitfähigkeit gemessen, die einen bestimmten Grenzwert übersteigt, beispielsweise einen Grenzwert von 15 mS/cm, so kann die Rückführung des Blutes in den Patienten unterbrochen und/oder eine Warnmeldung ausgegeben werden.

Vorliegend wird auch ein Verfahren offenbart zum Betrieb eines Dialysegerätes, wobei das Verfahren Schritte zur Ausführung der erfindungsgemäßen Verwendung umfasst.

Die erfindungsgemäße Verwendung kann also mittels eines herkömmlichen Dialysegerätes erfolgen, wobei dem zu reinigenden Blut vor Beginn und/oder während der Passage durch den Dialysator eine Lösung regel- und/oder steuerbar zudosierbar ist, so dass dem zu reinigenden Blut vor dem Beginn und/oder während der Passage durch den Dialysator ein oder mehrere in der Lösung enthaltene und natürlicherweise in Blutplasma vorkommende Ionen oder Salze davon zugesetzt werden, so dass das zu reinigende Blut zu Beginn und/oder während der Passage durch den Dialysator mindestens in Bezug auf eines dieser Ionen oder Salze davon eine Konzentration aufweist, die gegenüber der physiologischen Konzentration des Ions oder Salzes in Blutplasma erhöht ist.

Ein solches Dialysegerät umfasst in der Regel einen Dialysatkreislauf, einen Blutkreislauf und einen Dialysator für den Stoffaustausch zwischen zu reinigendem Blut des Blutkreislaufs und Dialysat des Dialysatkreislaufs.

Im Dialystkreislauf wird das Dialysat bewegt, gegen welches das zu reinigende Blut dialysiert werden soll. Dabei wird unter dem Begriff "Dialysatkreislauf" eine Leitungsanordnung verstanden in der das Dialysat aus einem Reservoir z. B. durch eine Pumpe derart gerichtet durch den Dialysator bewegt werden kann, so dass das Dialysat den Dialysator im Gegenstrom zum zu reinigenden Blut auf der dem Blut abgewandten Seite der Membran des Dialysators geführt wird. Das Dialysat nimmt durch die semipermeable Membran des Dialysators diffundierte Stoffe, wie z. B. Urämie-Toxine aber auch natürlicherweise im Blutplasma vorkommende Ionen oder Salze, auf und führt diese aus dem Dialysator ab. Nach der Passage durch den Dialysator kann das "verbrauchte" Dialysat abgeführt und ggf. in einem weiteren Behälter aufgefangen und gesammelt werden. Alternativ dazu kann das Dialysat dem Dialysatkreislauf für eine weitere Passage des Dialysators zugeführt werden.

Im Blutkreislauf wird das zu reinigende Blut nach der Entnahme (also "extrakorporal") bewegt. Dabei wird unter dem Begriff "Blutkreislauf" oder "extrakorporaler Blutkreislauf" eine Leitungsanordnung verstanden, in der das zu reinigende Blut dem Patienten entnommen wird und z. B. durch eine Pumpe derart gerichtet durch den Dialysator bewegt werden kann, so dass das zu reinigende Blut den Dialysator im Gegenstrom zum Dialysat auf der dem Dialysat abgewandten Seite der semipermeablen Membran des Dialysators geführt wird.

Nach der Passage durch den Dialysator wird das gereinigte Blut dem Patienten wieder zurückgeführt. Grundsätzlich ist es auch möglich, dass Dialysat und Blut im Dialysator in paralleler Flussrichtung bewegt werden, allerdings ist eine solche Anordnung in der Regel weniger effektiv als eine Anordnung im Gegenstromprinzip.

Dabei werden die Ionen oder Salze dem zu reinigenden Blut im Blutkreislauf an einer geeigneten Stelle nach der Entnahme und vor dem Eintritt des Blutes in den Dialysator und/oder im Dialysator zugeführt. Dazu kann das Dialysegerät einen zusätzlichen Zugang oder Anschluss im Blutkreislauf zwischen dem Einlass für das zu reinigende Blut und dem Eintritt in den Dialysator aufweisen. Das Dialysegerät kann ggf. ein Reservoir zur Aufnahme einer Lösung enthaltend ein oder mehrere natürlicherweise in Blutplasma vorkommende Ionen oder Salze davon aufweisen, wobei das Reservoir mit dem Blutkreislauf flüssigkeitsleitend verbunden ist. Zusätzlich kann das Dialysegerät eine Regel- und/oder Steuereinheit aufweisen zur gezielten Abgabe von Lösung aus dem Reservoir. In einer bevorzugten Ausführungsform ist die Regel- und/oder Steuereinheit derart ausgebildet, dass sich darüber zusätzlich auch Parameter des Dialysekreislaufs und/oder des Blutkreislaufes, wie z. B. die Flussgeschwindigkeit des zu reinigenden Blutes und/oder des Dialysats, durch einen Benutzer regeln und/oder steuern lassen.

Im Folgenden wird die Erfindung anhand von Ausführungsbeispielen näher erläutert.

Figuren:
- Figur 1: zeigt den Effekt von NaCl auf die Plasma-Proteinbindung unterschiedlicher Urämie-Toxine mittels Größenausschlusschromatographie, A: Plasma mit p-Cresol, 0,5 M NaCl, B: Plasma mit p-Hydroxyhippursäure, 0,5 M NaCl, C: Plasma mit Indoxylsulfat, 0,3 M NaCl.

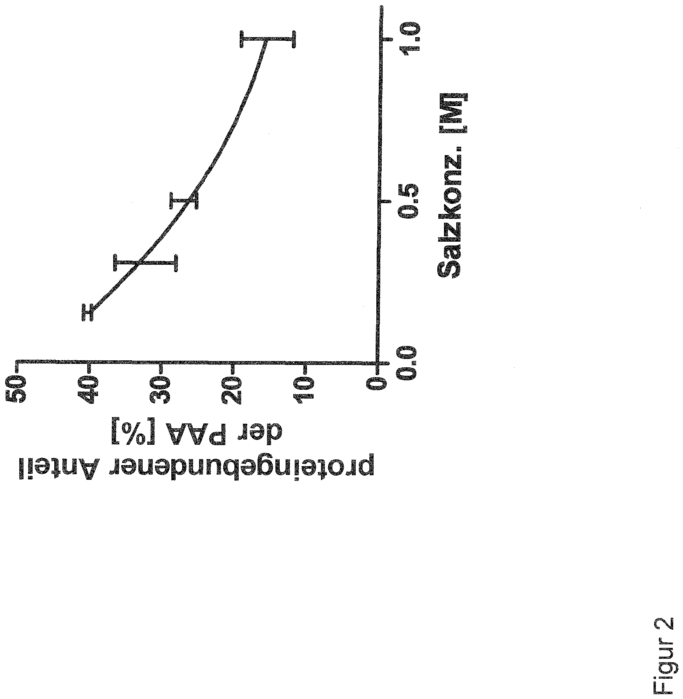

- Figur 2: zeigt den Anteil proteingebundener Phenylessigsäure (PAA) am Gesamtge-halt PAA in % bei steigenden NaCI-Konzentrationen.
- Figur 3: zeigt die Mengenverhältnisse an proteingebundener und freier PAA in Abhängigkeit der NaCI-Konzentration.
- Figur 4: zeigt den Anteil freien p-Cresylsulfates in % bei einem bestimmten Gesamtge-halt an p-Cresylsulfat in Abhängigkeit der NaCI-Konzentration.
- Figur 5: zeigt den Anteil freien Indoxylsulfat in % bei einem bestimmten Gesamtgehalt an Indoxylsulfat in Abhängigkeit der NaCI-Konzentration.
- Figur 6: zeigt die Konzentration von Indoxylsulfat bei Einsatz von in Indoxylsulfat in 1.) einer wässerigen NaCI-Lösung, 2.) in Hämofiltrat, 3.) in einer 4.5%igen Albuminlösung sowie 4.) in einer Lösung aus Hämofiltrat, in einer 4.5%igen Albuminlösung im Dialysat in Abhängigkeit des Porendurchmessers des verwendeten Hämodialysefilters.
- Figur 7: zeigt den Verlauf der Leitfähigkeit bei Infusion einer 5M NaCI-Lösung bei einer Dialysatflußrate Q_{D} von 500ml/min sowie unterschiedlichen Blutpumpengeschwindigkeiten (200ml/min bzw. 500 ml/min) an unterschiedlichen Stellen im Blutkreislauf mit Blod bag = Blut bei Aufnahme in den Blutkreislauf, Dialyzer in = unmittelbar vor dem Eintritt des Blutes in den Dialysator, Dialyzer out = unmittelbar nach dem Austritt aus dem Dialysator.
- Figur 8: zeigt den Verlauf der Leitfähigkeit bei Infusion einer 5M NaCI-Lösung bei einer Dialysatflußrate Q_{D} von 800ml/min sowie unterschiedlichen Blutpumpengeschwindigkeiten (200ml/min bzw. 500 ml/min) an unterschiedlichen Stellen im Blutkreislauf mit Blod bag = Blut bei Aufnahme in den Blutkreislauf, Dialyzer in = unmittelbar vor dem Eintritt des Blutes in den Dialysator, Dialyzer out = unmittelbar nach dem Austritt aus dem Dialysator.

### Beispiele:

### Beispiel 1: Nachweis des Effektes in vitro

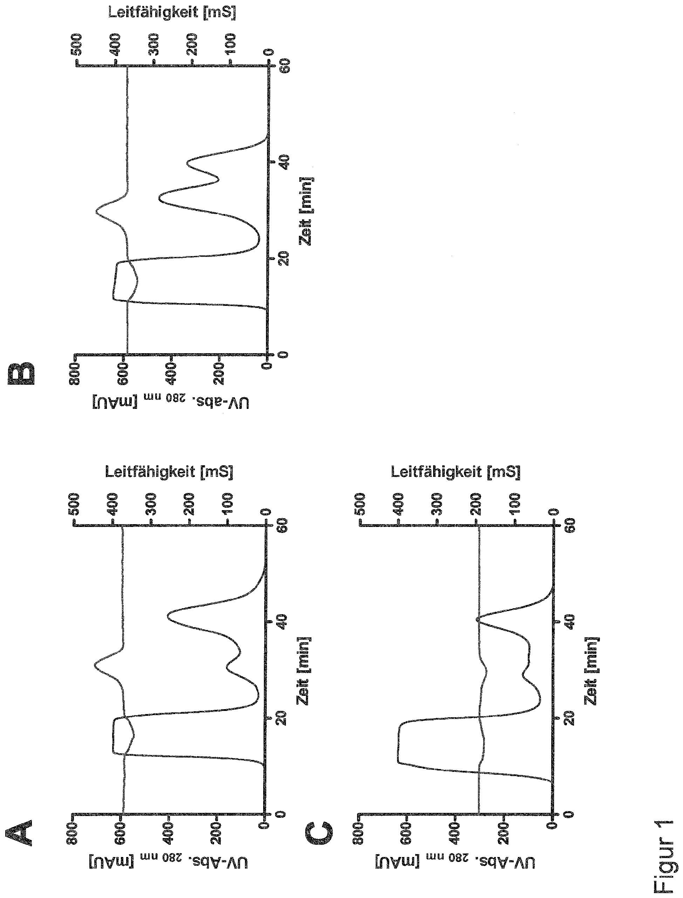

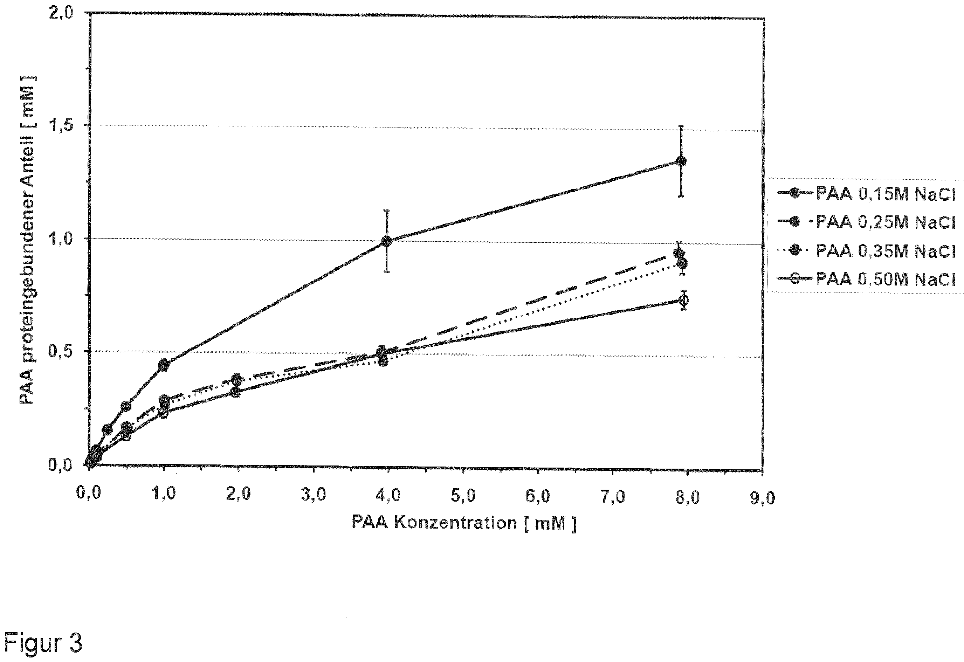

In in-vitro-Versuchsreihen wurde der Einfluss von Kochsalz auf den proteingebundenen Anteil von Urämietoxinen untersucht. Plasma gesunder Probanden wurde mit repräsentativen Toxinen aus der Gruppe der proteingebundenen, hydrophoben und aromatischen Urämietoxine versetzt und mit Hilfe chromatographischer Verfahren bis zur Homogenität aufgereinigt. Unter Verwendung von Größenausschlusschromatographie (SEC) wurden proteingebundene und freie Urämietoxine voneinander getrennt (siehe Figur 1). Verwendung fanden folgende Kochsalzkonzentrationen: 0,154 M (0,9 %) Kochsalz als physiologischer Wert und Ausgangspunkt für die weitere Untersuchung, 0,308 M (1,8 %), 0,5 M und 1 M Kochsalz. Die quantitative Bestimmung der Urämietoxinmenge erfolgte chromatographisch durch Vergleich mit Kalibrierkurven. Der Effekt ansteigender NaCI-Konzentration auf den Anteil protein-gebundener Phenylessigsäure als beispielhaftes aromatisches, nierdermolekulares Urämie-Toxin ist in Figur 3 gezeigt.

### Beispiel 2: Nachweis Effekt in Plasma

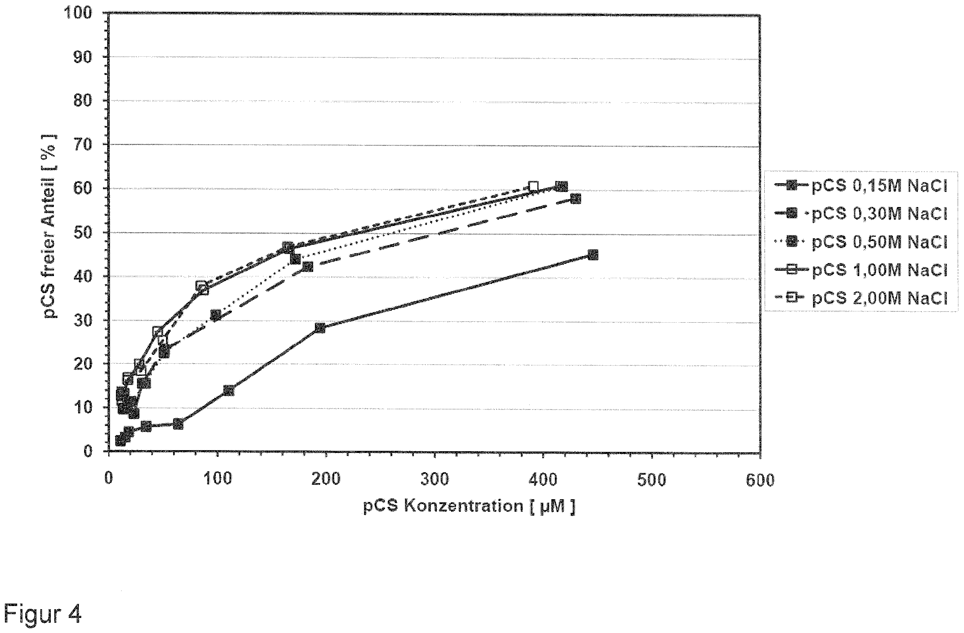

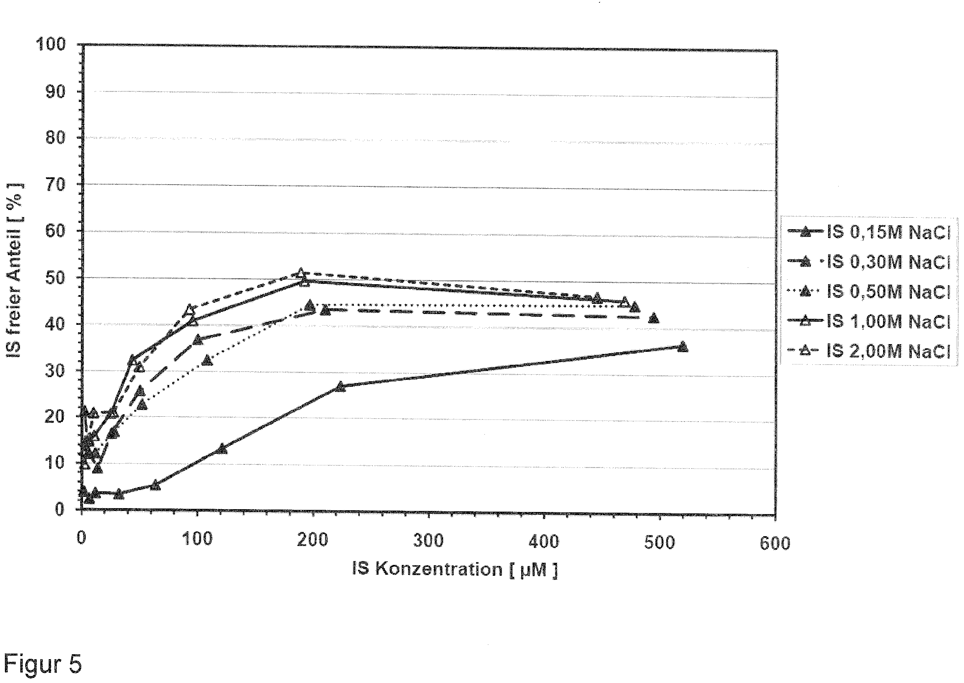

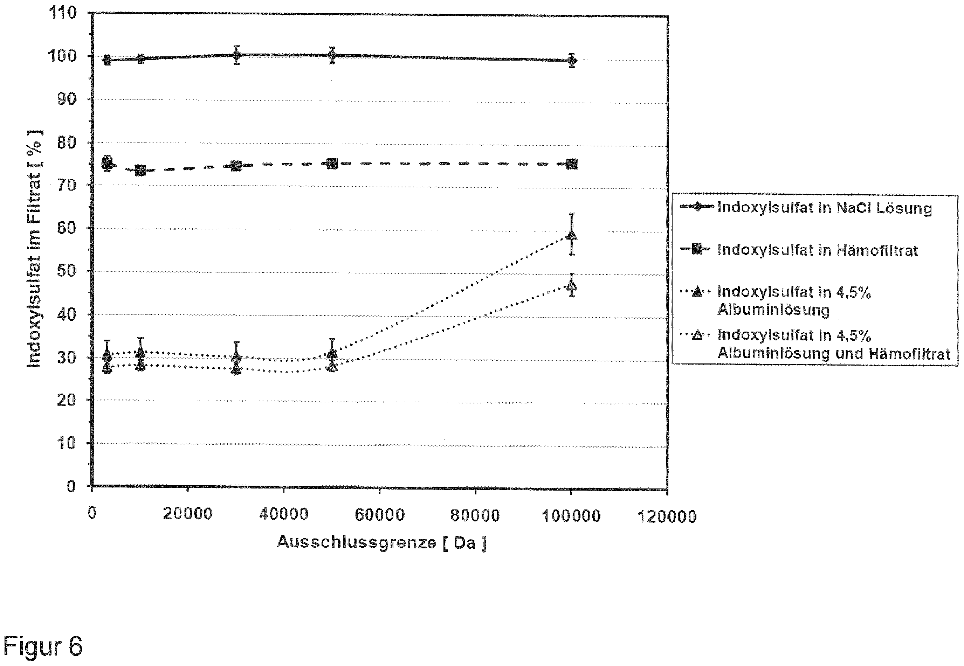

Um den proteingebundenen und freien Teil der Toxine in proteinhaltigen Medien schneller als über die Größenausschlußchromatographie quantifizieren zu können, wurde eine Methode entwickelt, die einerseits über ein Membranfiltrationsverfahren den freien Anteil abtrennt, und in der Hitze das Protein ausfällt, so dass der vorher proteingebundene Anteil nun über RP-HPLC bestimmt werden kann. Im Experiment, das in Figur 3 wiedergegeben ist, wurden steigende Mengen PAA zu Plasma dazugegeben und der proteingebundene Anteil nach Filtration der freien PAA mittels HPLC bestimmt. Dabei markiert die Kurve für 0.15M NaCl die physiologische lonenstärke im Plasma. Die PAA-Konzentration in uremischen Plasma liegt bei etwa 4 mM. Erhöht man durch Zugabe von Kochsalz die lonenstärke auf ≥ 0.25 M NaCl, sinkt der proteingebundene Anteil um etwa 50% ab (siehe Datenpunkte bei 4 mM PAA). D. h., dass bei höheren lonenstärken die Proteinbindung von PAA im Plasma abnimmt. Wie in Figuren 4 und 5 dargestellt, kann dieser Effekt auch für die Urämie-Toxine p-Cresylsulfat und Indoxylsulfat beobachtet werden. Der Effekt ist also nicht auf ein bestimmtes Urämie-Toxin beschränkt, sondern kann für verschiedene proteingebundene Urämie-Toxine reproduziert werden. In Figur 6 ist die nominale Porengröße des Filters variiert worden, mit dem der freie bzw. der proteingebundene Anteil des Urämie-Toxins iS (Indoxylsulfat) getrennt worden ist. Erst bei einer nominalen Pore von > 50.000 gehen nennenswerte Anteile an proteingebundenem Toxin in das Filtrat über. Dies spricht dafür, dass das Toxin auch im Plasma vorwiegend an HSA gebunden vorliegt.

### Beispiel 3: Verwendung am Beispiel einer Hämodiafiltration

Zur Gewährleistung einer physiologischen NaCI-Konzentration am Dialysatorausgang, wurde bei konstanter Infusion einer 5 M wässerigen NaCI-Lösung der Fluss der Blutpumpe und der Dialysatpumpe variiert. Der Fluss der 5 M wässerigen NaCI-Lösung wurde jeweils so variiert, dass sich am Dialysatoreingang eine effektive NaCI-Konzentration von 0,5 M einstellt. Die jeweils resultierende Leitfähigkeit am Dialysatorausgang wurde bestimmt.

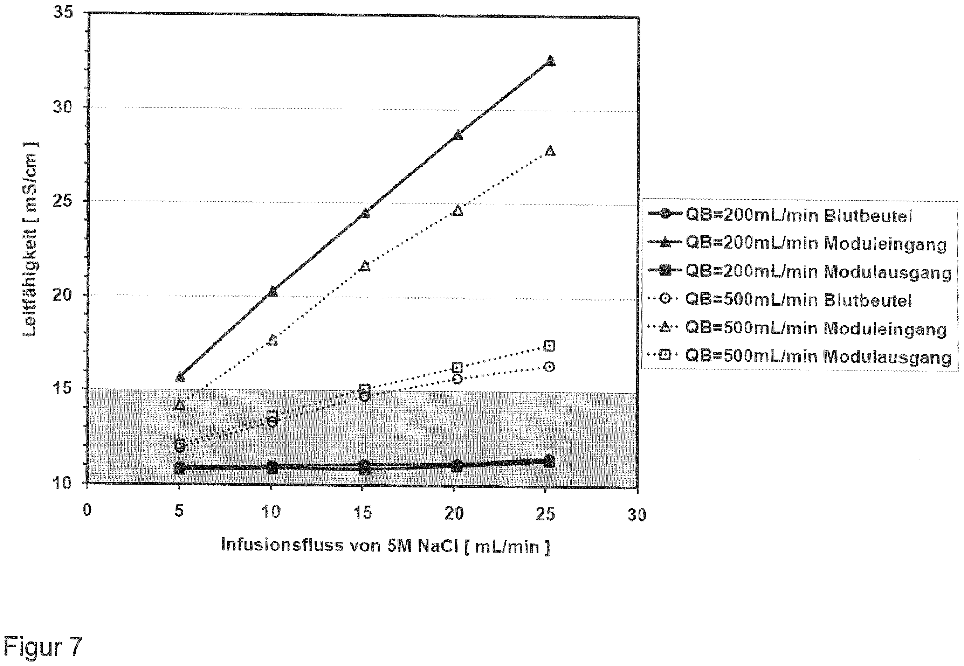

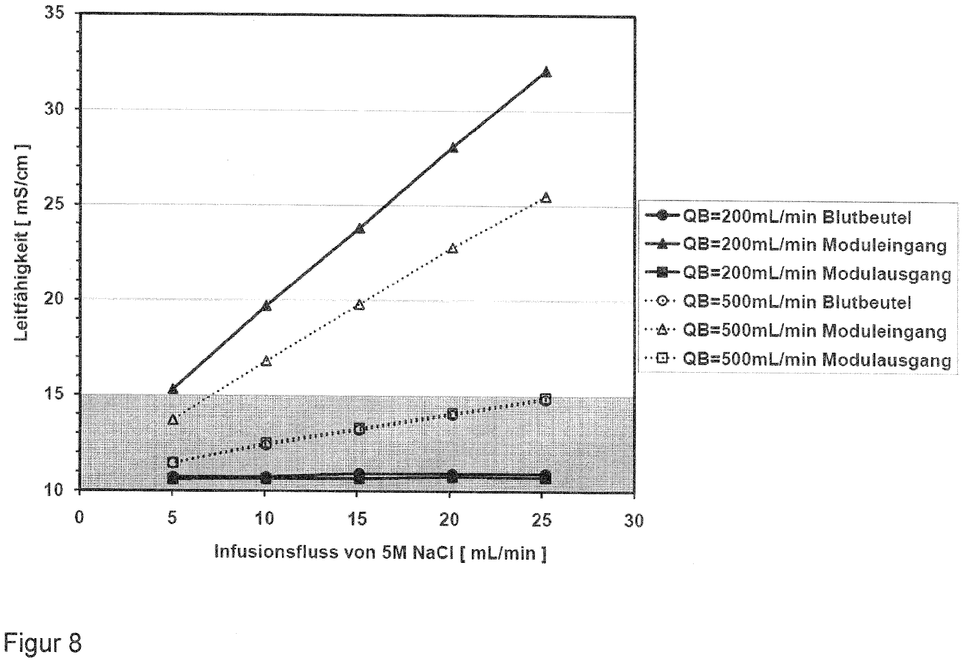

In der Figur 7 ist der Verlauf der Leitfähigkeit bei Infusion einer 5M NaCI-Lösung bei unterschiedlichen Blutpumpengeschwindigkeiten gezeigt. Es zeigt sich, dass durch Variation der Flussgeschwindigkeiten am Dialyseausgang problemlos Leitfähigkeiten wie im unbehandelten Plasma eingestellt werden können. Der schraffierte Bereich unterhalb einer Leitfähigkeit von 15 mS/cm und oberhalb einer Leitfähigkeit von 10 mS/cm gibt den Bereich der Leitfähigkeit an, den gereinigtes Blut aufweisen kann um gefahrlos an den Patienten zurückgeführt werden zu können. Ein Weg die lonenstärke am Ende des Dialysators auch bei sehr hohen Blutflußraten niedrig zu halten, stellt die Erhöhung der Dialysatflußrate dar. Dies ist aus dem Vergleich der Figuren 7 und 8 zu sehen. Während in Figur 7 die Dialysatflußrate Q_{D} 500ml/min betrug, so ist in Figur 8 eine Dialysatflußrate von Q_{D} 800ml/min verwendet worden.

## Patentansprüche

1. Ionen oder Salze davon zur Verwendung in einem Verfahren zur Hämodialyse oder Hämodiafiltration mittels eines Dialysators zum Stoffaustausch, **dadurch gekennzeichnet, dass** dem zu reinigenden Blut vor dem Beginn und/oder während der Passage durch den Dialysator ein oder mehrere natürlicherweise in Blutplasma vorkommende Ionen oder Salze davon zugesetzt werden, so dass das zu reinigende Blut zu Beginn der Passage durch den Dialysator mindestens in Bezug auf eines dieser Ionen oder Salze davon eine Konzentration aufweist, die gegenüber der physiologischen Konzentration des Ions oder Salzes in Blutplasma um einen Faktor ≥ 1,5 bis 10 erhöht ist,
wobei es sich bei den Ionen oder Salzen davon um das Ion Na⁺ oder das Salz NaCl handelt.

2. Ionen oder Salze davon nach einem der vorhergehenden Ansprüche, wobei dem zu reinigenden Blut vor dem Beginn der Passage durch den Dialysator das Ion Na⁺ oder das Salz NaCl zugesetzt werden, so dass das zu reinigende Blut zu Beginn der Passage durch den Dialysator mindestens in Bezug auf das Ion Na⁺ oder das Salz NaCl eine Konzentration aufweist, die gegenüber der physiologischen Konzentration des Ions oder Salzes in Blutplasma um einen Faktor von ≥ 2 bis 8 erhöht ist.

3. Ionen oder Salze davon nach einem der vorhergehenden Ansprüche, wobei das zu reinigende Blut zu Beginn der Passage durch den Dialysator eine NaCI-Konzentration aufweist von ≥ 0,25 M, bevorzugt von ≥ 0,3 M, besonders bevorzugt von ≥ 0,25 M bis 1,5 M, ganz besonders bevorzugt von ≥ 0,3 M bis 1 M.

4. Ionen oder Salze davon nach einem der vorhergehenden Ansprüche, wobei das zu reinigende Blut vor dem Beginn der Passage durch den Dialysator mit einer Elektrolytlösung versetzt wird, die in Bezug auf das Ion Na⁺ oder das Salz NaCl eine Konzentration aufweist, die gegenüber der physiologischen Konzentration in Blutplasma erhöht ist, während die Elektrolytlösung zusätzlich andere natürlicherweise in Blutplasma vorkommende Ionen oder Salze davon in physiologischer Konzentration enthält.

5. Ionen oder Salze davon nach einem der vorhergehenden Ansprüche, wobei der Dialysator eine semipermeable Membran aufweist mit einer Größenausschlussgrenze, die geeignet ist, die vor dem Beginn der Passage durch den Dialysator dem zu reinigenden Blut zugesetzten Ion Na⁺ oder Salz NaCl durch die Membran passieren zu lassen, bevorzugt mit einer Größenausschlussgrenze von ≥ 10.000 Da.

6. Ionen oder Salze davon nach einem der vorhergehenden Ansprüche, wobei das Blut nach der Passage durch den Dialysator und vor einer Rückführung in den Patienten daraufhin überprüft wird, ob ausgewählte Parameter des Blutes vorher festgelegte Grenzwerte überschreiten und wobei, sollte ein solcher Grenzwert überschritten sein, die Rückführung des Blutes in den Patienten unterbunden wird.

7. Ionen oder Salze davon nach Anspruch 6, wobei die Parameter die Leitfähigkeit umfassen.

8. Ionen oder Salze davon nach Anspruch 7, wobei eine Überschreitung eines Grenzwertes vorliegt, wenn die Leitfähigkeit des Blutes eine Leitfähigkeit von 15 mS/cm übersteigt.

## Claims

1. The ions or salts thereof for use in a method for hemodialysis or hemodiafiltration by means of a dialyzer for exchanging substances, **characterized in that** one or a plurality of ions or salts thereof naturally occurring in the blood plasma are added to the blood to be purified before the start and/or during the passage through the dialyzer such that the blood to be purified, at the start of the passage through the dialyzer, has a concentration that is increased with respect to the physiological concentration of the ion or salt in blood plasma by a factor of ≥ 1.5 to 10 at least in relation to one of these ions or salts thereof,
wherein the ions or salts thereof are the Na⁺ ion or the NaCl salt.

2. The ions or salts thereof according to any of the preceding claims, wherein the Na⁺ ion or the NaCl salt is added to the blood to be purified before the start of the passage through the dialyzer such that the blood to be purified, at the start of the passage through the dialyzer, has a concentration that is increased with respect to the physiological concentration of the ion or salt in blood plasma by a factor of ≥ 2 to 8 at least in relation to the Na⁺ ion or the NaCl salt.

3. The ions or salts thereof according to any of the preceding claims, wherein the blood to be purified, at the start of the passage through the dialyzer, has an NaCl concentration of ≥ 0.25 M, preferably of ≥ 0.3 M, especially preferably of from ≥ 0.25 M to 1.5 M, or very especially preferably of from ≥ 0.3 M to 1 M.

4. The ions or salts thereof according to any of the preceding claims, wherein the blood to be purified, before the start of passage through the dialyzer, is added to an electrolyte solution that has a concentration in relation to the Na⁺ ion or the NaCl salt that is increased with respect to the physiological concentration in blood plasma, while the electrolyte solution additionally contains other ions or salts thereof that occur naturally in blood plasma in physiological concentration.

5. The ions or salts thereof according to any of the preceding claims, wherein the dialyzer has a semi-permeable membrane with a size exclusion limit, which is suitable, before the start of the passage through the dialyzer, for allowing the passage of the Na⁺ ion or NaCl salt added to the blood to be purified through the membrane, preferably with a size exclusion limit of ≥ 10,000 Da.

6. The ions or salts thereof according to any of the preceding claims, wherein the blood is checked after passage through the dialyzer and before a return into the patient as to whether selected parameters of the blood exceed previously specified limits and wherein, if such a limit has been exceeded, the return of the blood to the patient is suppressed.

7. The ions or salts thereof according to claim 6, wherein the parameters comprise the conductivity.

8. The ions or salts thereof according to claim 7, wherein an exceeding of a limit exists when the conductivity of the blood exceeds a conductivity of 15 mS/cm.

## Revendications

1. Ions, ou sels de ceux-ci, pour l'utilisation dans un procédé d'hémodialyse ou d'hémodiafiltration au moyen d'un dialyseur pour l'échange de substances, **caractérisés en ce que** au sang à purifier est ajouté, avant le début et/ou pendant le passage à travers le dialyseur, un ou plusieurs ions, ou sels de ceux-ci, existant naturellement dans le plasma sanguin, de telle sorte que le sang à purifier présente, au début du passage à travers le dialyseur, au moins par rapport à l'un de ces ions, ou sels de ceux-ci, une concentration qui est augmentée d'un facteur de ≥ 1,5 à 10 par rapport à la concentration physiologique en ion ou en sel dans le plasma sanguin,
où il s'agit, pour les ions, ou sels de ceux-ci, de l'ion Na⁺ ou du sel NaCl.

2. Ions, ou sels de ceux-ci, selon l'une quelconque des revendications précédentes, le sang à purifier étant additionné, avant le début du passage à travers le dialyseur, de l'ion Na⁺ ou du sel NaCl de telle sorte que le sang à purifier, au début du passage à travers le dialyseur présente, au moins par rapport à l'ion Na⁺ ou au sel NaCl, une concentration qui est augmentée d'un facteur ≥ 2 à 8 par rapport à la concentration physiologique en ion ou en sel dans le plasma sanguin.

3. Ions, ou sels de ceux-ci, selon l'une quelconque des revendications précédentes, le sang à purifier présentant, au début du passage à travers le dialyseur, une concentration en NaCl ≥ 0,25 M, de préférence ≥ 0,3 M, de manière particulièrement préférée de ≥ 0,25 M à 1,5 M, de manière tout particulièrement préférée de ≥ 0,3 M à 1 M.

4. Ions, ou sels de ceux-ci, selon l'une quelconque des revendications précédentes, au sang à purifier étant ajouté, avant le début du passage à travers le dialyseur, une solution électrolytique, qui présente, par rapport à l'ion Na⁺ ou au sel NaCl, une concentration qui est augmentée par rapport à la concentration physiologique dans le plasma sanguin, alors que la solution électrolytique contient en outre d'autres ions, ou sels de ceux-ci, qui existent naturellement dans le plasma sanguin, à la concentration physiologique.

5. Ions, ou sels de ceux-ci, selon l'une quelconque des revendications précédentes, le dialyseur présentant une membrane semi-perméable dotée d'une limite d'exclusion de taille qui est appropriée pour laisser passer, avant le début du passage à travers le dialyseur, l'ion Na⁺ ou le sel NaCl ajouté au sang à purifier, à travers la membrane, de préférence pourvue d'une limite d'extrusion de taille ≥ 10 000 Da.

6. Ions, ou sels de ceux-ci, selon l'une quelconque des revendications précédentes, le sang étant testé après le passage à travers le dialyseur et avant le retour au patient pour déterminer si des paramètres sélectionnés du sang dépassent des valeurs limites fixées au préalable et, si une telle valeur limite est dépassée, le retour du sang au patient étant empêché.

7. Ions, ou sels de ceux-ci, selon la revendication 6, les paramètres comprenant la conductibilité.

8. Ions, ou sels de ceux-ci, selon la revendication 7, un dépassement d'une valeur limite existant lorsque la conductibilité du sang dépasse une conductibilité de 15 mS/cm.
